Europäisches Patentamt

European Patent Office

Office européen des brevets

(11) Publication number: **0 246 998**
**A2**

# (12) EUROPEAN PATENT APPLICATION

(21) Application number: **87630093.0**

(22) Date of filing: **19.05.87**

(51) Int. Cl.⁴: **A 61 M 29/02**
**A 61 F 2/06**

(30) Priority: **21.05.86 US 865402**

(43) Date of publication of application:
**25.11.87 Bulletin 87/48**

(84) Designated Contracting States:
**DE ES FR GB IT SE**

(71) Applicant: **ZETA LTD.**
**Atidim Scientific Industries Park Dvora Hanevia St-**
**Tel Aviv 61 101 (IL)**

(72) Inventor: **Porath-Furedi, Asher**
**Kubovy St. 10**
**Ramat Dania Jerusalem (IL)**

(74) Representative: **Schmitz, Jean-Marie et al**
**OFFICE DENNEMEYER S.à.r.l. P.O. Box 1502**
**L-1015 Luxembourg (LU)**

(54) Cardiac balloon catheter.

(57) A cardiac balloon catheter for opening an occlusion in a blood vessel comprises a split sleeve received over the balloon when deflated so as to be moved therewith to the occlusion. At the occlusion, the split sleeve is expanded to a larger diameter by the inflation of the balloon. The sleeve is self-supporting in its expanded condition, and remains in place in engagement with the blood vessel wall upon the deflation and removal of the balloon catheter from the blood vessel. The catheter may also include a bypass conduit providing a flow path for the blood bypassing the balloon when the balloon is inflated.

FIG. 1

EP 0 246 998 A2

**Description**

CARDIAC BALLOON CATHETER

The present invention relates to cardiac balloon catheters, and particularly to such catheters which include a balloon carried by the catheter tube and inflatable to open an occluded blood vessel.

Cardiac vascular diseases involving the partial or total occlusion or blocking of a pulmonary blood vessel are today one of the main causes of death. The total or partial occlusion of a blood vessel may result from the formation of atherosclerotic plaque on the inner walls of the blood vessel; or from hemorrhaging into the atherosclerotic plaque within the blood vessel wall, increasing the wall thickness accompanied by inward swelling; or from the formation of a blood clot clogging the blood vessel.

Balloon catheterization techniques have recently been developed for opening partially or totally occluded blood vessels. According to this technique, a cardiac balloon catheter comprising an elongated, flexible catheter tube is passed through the blood vessel to position a balloon carried by the catheter tube at the location of the occlusion; and the balloon is inflated to engage the occlusion and to press it inwardly in order to widen the passage through the blood vessel. The balloon is then deflated and removed with the catheter.

An advantage of the the balloon catheterization technique is that it avoids bypass surgery, which heretofore was generally required in order to provide a bypass for the occluded portion of the blood vessel. However, the widening of the artery by the inflation of the balloon is frequently of a very short duration. Sometimes it disappears immediately after the deflation of a balloon, and other times at a relatively short time thereafter. Thus, the relief, if produced, may be very short-lived.

In addition, balloon catheterization suffers from the danger that when the balloon is inflated, the passage of the blood is completely shut-off. This is particularly dangerous if the occlusion was only a partial occlusion, and not a complete occlusion. Thus, if the treated condition involved a complete occlusion, then obviously there must have been a secondary supply of blood to the heart muscles before the inflation of the balloon, and therefore the supply will still remain in effect when the balloon is inflated. However, if the treated condition was only a partial occlusion, then the inflation of the balloon during the balloon catheterization treatment may result in a complete stoppage of the blood to the portion of the heart muscle, and may cause severe chest pains (angina) and possibly even a myocardial infarction.

An object of the present invention is to provide a cardiac balloon catheter having advantages in the above respects.

According to the present invention, there is provided a cardiac balloon catheter for opening an occlusion in a blood vessel, comprising an elongated, flexible catheter tube receivable within the blood vessel, and a balloon carried by the catheter tube and inflatable to open the occlusion in the blood vessel; characterized in that the catheter further includes a sleeve received over the balloon when deflated for movement therewith to the occlusion in the blood vessel; the sleeve being longitudinally split so as to be expansible from a small diameter to a large diameter upon the inflation of the balloon, and being self-supporting in its larger-diameter condition to support the wall of the blood vessel upon the deflation of the balloon and the removal of the balloon catheter from the blood vessel.

According to another feature in the described preferred embodiment, the catheter further includes a bypass conduit having its opposite ends communicating with the outer faces of the catheter tube on the opposite sides of the balloon to provide a flow path for the blood bypassing the balloon when the balloon is inflated.

Further features and advantages of the invention will be apparent from the description below.

The invention is herein described, by way of example only, with reference to the accompanying drawings, wherein:

Figs. 1-3 are longitudinal sectional views illustrating three types of cardiac balloon catheters constructed in accordance with the present invention;

Fig. 4 is a longitudinal sectional view illustrating a further cardiac balloon catheter, together with a self-supporting sleeve, constructed in accordance with the present invention; and

Figs. 5 and 6 are end views illustrating two types of self-supporting sleeves which may be used in the catheter of Fig. 3; and

Fig. 7 is a three-dimensional view illustrating a further type of self-supporting sleeve that may be used.

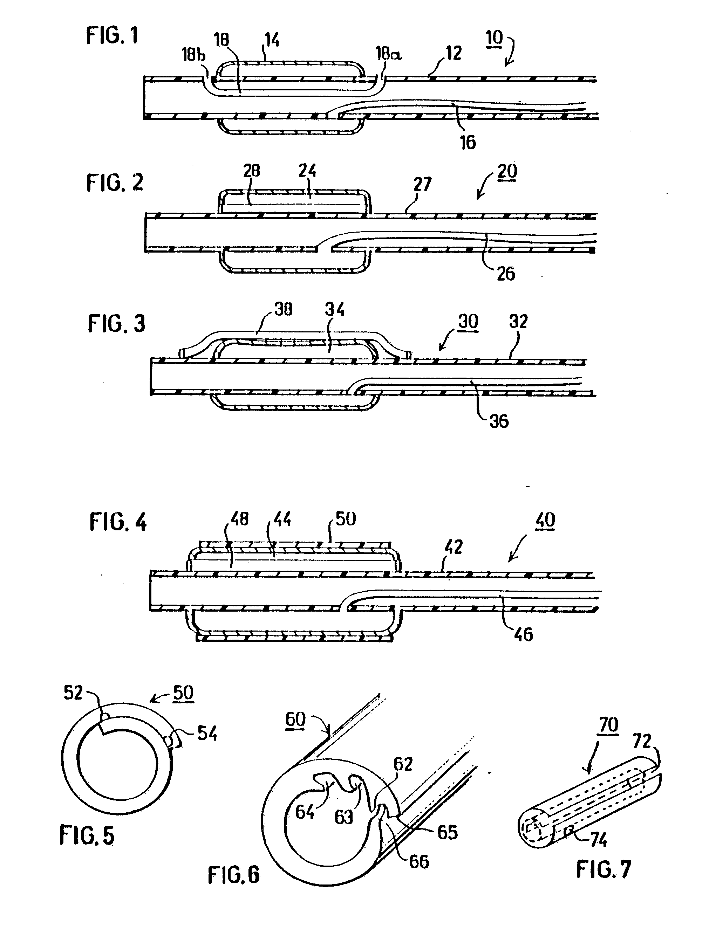

Fig. 1 illustrates a form of cardiac balloon catheter, therein designated 10, comprising an elongated, flexible catheter tube 12 which is receivable within a blood vessel, and a balloon 14 carried by the catheter tube and inflatable by a tubelet 16 to open the occluded blood vessel. The catheter is provided with a bypass conduit 18 passing through the catheter tube 12 and having its opposite ends 18a, 18b communicating with the outer face of the catheter tube on the opposite sides of the balloon 14. Thus, conduit 18 provides a flow path for the blood bypassing the balloon when the balloon is inflated, thereby preventing the complete blockage of the blood flow when the balloon is inflated.

Fig. 2 illustrates a variation, wherein the catheter balloon, therein designated 20, also includes a catheter tube 22, a balloon 24 inflatable by a tubelet 26, and a bypass conduit 28; but in the variation illustrated in Fig. 2, the bypass conduit 28 passes through the balloon 24, rather through the catheter tube 22 as in Fig. 1.

Fig. 3 illustrates a further variation, wherein the balloon catheter, therein designated 30, also includes a catheter tube 32, a balloon 34 inflated by a tubelet 36, and a bypass conduit 38; but in the variation illustrated in Fig. 3, the bypass conduit 38 is supported on the outer face of the balloon 34.

Fig. 4 illustrates a balloon catheter of the type illustrated in Fig. 2 but including a sleeve which is introduced into the blood vessel with the catheter. The sleeve remains in the blood vessel after removal of the catheter in order to support the blood vessel wall in the opened condition produced by the inflation of the balloon. While the sleeve is shown in Fig. 4 as included in a balloon catheter of the type illustrated in Fig. 2, it will be appreciated it could also be included in other type catheters, for example that illustratd in Fig. 1.

Thus, the catheter 40 illustrated in Fig. 4 includes a catheter tube 42, a balloon 44 inflated by a tubelet 46, and a bypass conduit 48 providing a flow path for the blood bypassing the balloon when the balloon is inflated. For purposes of example, bypass conduit 48 is illustrated of the Fig. 2 type, namely of the type passing through the balloon.

Catheter 40 illustrated in Fig. 4, further includes a split sleeve 50 received over the balloon when deflated for introduction therewith into the blood vessel in alignment with the occlusion. Split sleeve 50 is of rigid elastic material, such as a biocompatible plastic. The split sleeve is of a small diameter when introduced with the catheter and balloon into the blood vessel, but is expansible to a larger diameter upon the inflation of the balloon. When so expanded, the sleeve is self-supporting in its larger-diameter condition in engagement with the inner wall of the blood vessel and remains in place upon the deflation of the balloon and the removal of the balloon with the catheter from the blood vessel.

Fig. 5 illustrates one example of a construction that may be used for split sleeve 50. In this example, the split sleeve is made of elastic material and is configured such that, in its relaxed condition, it assumes its larger-diameter for supporting the blood vessel wall after removal of the catheter. Sleeve 50, however, is releasably retained, by releasable retainer means, in its smaller diameter condition during its insertion into the blood vessel with the catheter. In the example illustrated in Fig. 5, the releasable retainer means comprises a first rib or projection 52 formed at one end of the split sleeve receivable within a correspondingly-figured recess formed adjacent to the opposite end of the sleeve, and a second rib or projection 54 formed adjacent to the first end of the sleeve and receivable within a correspondingly-shaped recess formed at the opposite end of the sleeve. Projections 52, 54, when received in their respective recesses, are sufficient to retain sleeve 50 under stress in its smaller-diameter condition as illustrated in Fig. 5.

This is the condition of the sleeve when inserted with the ballon and its catheter into the blood vessel and manipulated to the location of the occlusion. When the sleeve is properly located in alignment with the occlusion, the balloon is inflated as described above. This applies sufficient force to the inner surface of the split sleeve 50 to cause its projections 52, 54 to unseat from their respective recesses, whereupon the split sleeve snaps-over to its larger-diameter condition pressing against the inner wall of the blood vessel. The balloon may then be deflated and removed with the catheter, leaving the split sleeve 50 supporting itself and also supporting the inner wall of the blood vessel in the relaxed, larger-diameter condition of the split sleeve.

Fig. 6 illustrates a variation in the construction of the split sleeve, therein designated 60. In this variation, the split sleeve is of its smaller diameter in its relaxed condition, and includes interlocking elements locking it in its larger-diameter condition when expanded by the inflation of the balloon. Thus, sleeve 60 is formed with a plurality of recesses, 62, 63, 64, spaced inwardly from one end 65 of the split, and adapted to receive the opposite end 66 of the split when the sleeve is expanded by the inflation of the balloon. The recesses are shaped such that end 66 may be moved into and out of a recess in the expanding direction of the sleeve, but not in the opposite direction. Thus, the sleeve is locked in its expanded condition.

The split sleeve 60 illustrated in Fig. 6 is capable of being expanded to and locked in a plurality of different outer diameters, according to the degree of inflation of the balloon and the dimensions of the blood vessel receiving it. The recesses could cooperate wtih a rib or projection formed adjacent to the end of the sleeve, rather than with the end itself.

Fig. 7 illustrates a still further variation in the structure of the self-supporting sleeve, therein designated 70. This sleeve is also split, as shown at 72, but is made of a semi-rigid, non-elastic material capable of enlarging its diameter upon the deflation and removal of the balloon. As one example, the sleeve may be of aluminum foil coated on both sides by a biocompatible material, such as silicone.

Preferably, the split sleeve 50, 60 or 70 (of Figs. 5, 6 and 7, respectively) includes a hole at one end, as shown at 74 in Fig. 7, to facilitate its removal if and when this should become necessary.

While the split sleeve is illustrated as included in a balloon catheter of the type illustrated in Fig. 2, it will be appreciated that it could be used with other types of balloon catheters.

## Claims

1. A cardiac ballon catheter for opening an occlusion in a blood vessel, comprising an elongated, flexible catheter tube receivable within the blood vessel, and a balloon carried by the catheter tube and inflatable to open the occlusion in the blood vessel; characterized in that said catheter further includes a sleeve received over said balloon when deflated for movement therewith to said occlusion in the blood vessel; said sleeve being longitudinally split so as to be expansible from a small diameter to a large diameter upon the inflation of the balloon, and being self-supporting in its larger-diameter condition to support the wall of the blood vessel upon the deflation of the balloon and the removal of the balloon catheter from the blood vessel.

2. The catheter according to Claim 1, wherein said split sleeve is of an elastic material and is of its larger diameter when in its relaxed condition, said split sleeve including releasable retainer means for retaining it in its stressed smaller-diameter condition until the inflation of the balloon releases it for expansion to its larger-diameter condition.

3. The catheter according to Claim 2, wherein said releasable retainer means comprises a projection at one end of the split sleeve and receivable within a recess adjacent to the other end of the split sleeve when the split sleeve is in its stressed smaller-diameter condition.

4. The catheter according to Claim 1, wherein said split sleeve is of an elastic material and is of its smaller diameter in its relaxed condition, said sleeve including locking means for locking the sleeve in its larger-diameter condition when expanded by the inflation of the balloon.

5. The catheter according to Claim 4, wherein said locking means comprises one or more recesses formed in an inner surface of said split sleeve and spaced inwardly from one end thereof, which recesses are adapted to receive the opposite edge of the split sleeve when the split sleeve is expanded to its larger diameter by the inflation of the balloon.

6. The catheter according to Claim 1, wherein said split sleeve is of a semi-rigid, non-elastic material capable of enlarging its diameter upon the inflation of the balloon and retaining its larger diameter upon the deflation and removal of athe balloon.

7. The catheter according to Claim 1, wherein said split sleeve includes a hole therethrough to facilitate its removal.

8. The catheter according to any one of Claims 1-7, wherein said catheter further includes a bypass conduit having its opposite ends communicating with the outer faces of the catheter tube on the opposite sides of the balloon to provide a flow path for the blood bypassing said balloon when the balloon is inflated.

9. The catheter according to Claim 8, wherein said bypass conduit passes through said balloon.

10. The catheter according to Claim 8, wherein said bypass conduit passes through said balloon.

FIG. 1

FIG. 2

FIG. 3

FIG. 4

FIG. 5

FIG. 6

FIG. 7